(19) Europäisches Patentamt
European Patent Office
Office européen des brevets

(11) Numéro de publication: **0 363 255 A1**

# (12) DEMANDE DE BREVET EUROPEEN

(21) Numéro de dépôt: **89402648.3**

(22) Date de dépôt: **27.09.89**

(51) Int. Cl.[5]: **C07H 15/252 , C07D 309/10 , C07C 255/13 , A61K 31/70**

(30) Priorité: **28.09.88 FR 8812658**

(43) Date de publication de la demande: **11.04.90 Bulletin 90/15**

(84) Etats contractants désignés: **AT BE CH DE ES FR GB GR IT LI LU NL SE**

(71) Demandeur: **LABORATOIRES HOECHST S.A.**
**TOUR ROUSSEL HOECHST, 1 Terrasse Bellini**
**F-92800 Puteaux(FR)**

Demandeur: **BEHRINGWERKE Aktiengesellschaft**
**Postfach 1140**
**D-3550 Marburg 1(DE)**

(72) Inventeur: **Renoux, Brigitte**
**Liniers**
**F-86800 Saint Julien l'Ars(FR)**
Inventeur: **Gesson, Jean-Pierre**
**"La Germonière" Montamise**
**F-86360 Chasseneuil du Poitou(FR)**
Inventeur: **Jacquesy, Jean-Claude**
**46 rue du Planty**
**F-86180 Buxerolles(FR)**
Inventeur: **Kraemer, Hans Peter**
**Birkenweg 16**
**D-3550 Marburg(DE)**

(74) Mandataire: **Orès, Bernard et al**
**Cabinet ORES 6, Avenue de Messine**
**F-75008 Paris(FR)**

(54) **Nouveaux analogues de la désoxy-4 daunosamine, procédé de préparation, anthracyclines obtenues à l'aide de ces analogues et utilisation desdites anthracyclines en tant que médicaments.**

(57) La présente invention est relative à de nouveaux analogues de la désoxy-4 daunosamine, à leur procédé de préparation, à des anthracyclines obtenues à l'aide de ces analogues et à l'utilisation desdites anthracyclines en tant que médicaments.

Les analogues de la désoxy-4 daunosamine, correspondent à la formule générale 1, ci-après :

EP 0 363 255 A1

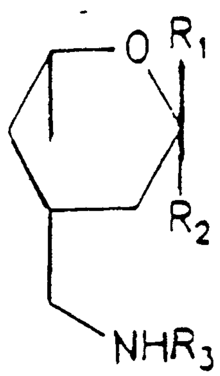

(1)

# NOUVEAUX ANALOGUES DE LA DESOXY-4 DAUNOSAMINE, PROCEDE DE PREPARATION, ANTHRACYCLINES OBTENUES A L'AIDE DE CES ANALOGUES ET UTILISATION DESDITES ANTHRACYCLINES EN TANT QUE MEDICAMENTS.

La présente invention est relative à de nouveaux analogues de la désoxy-4 daunosamine, à leur procédé de préparation, à des anthracyclines obtenues à l'aide de ces analogues et à l'utilisation desdites anthracyclines en tant que médicaments.

Dans la série des anthracyclines, médicaments utilisés cliniquement pour le traitement de diverses formes de cancer, la modification de la partie osidique se traduit généralement par l'obtention d'un spectre d'activité différent et par une variation de la toxicité de ces molécules. Cette méthode permet ainsi d'obtenir de nouveaux composés semisynthétiques de grand intérêt thérapeutique pour des formes de cancer montrant une résistance (naturelle ou acquise) aux anthracyclines les plus couramment utilisées comme la daunorubicine 2a ou la doxorubicine 2b.

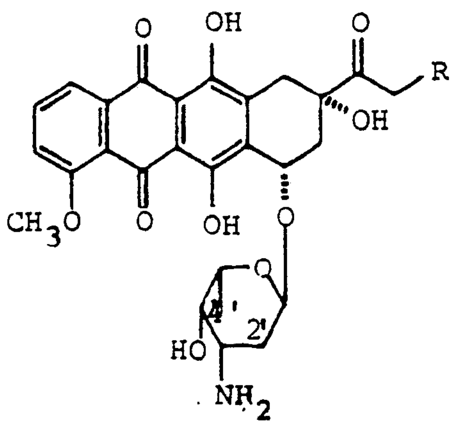

2a R = H

2b R = OH

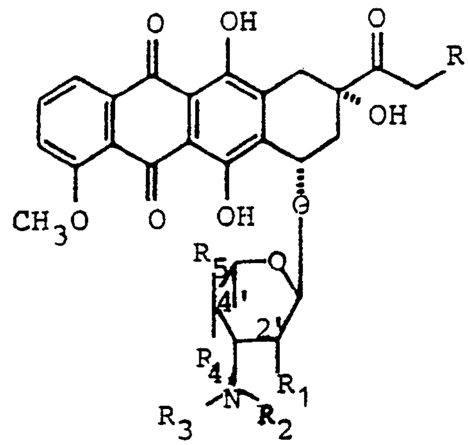

3 R = H

4 R = OH

Des modifications structurales (voir F. ARCAMONE) Doxorubicin, Anticancer Antibiotics, Academic Press, 1982) ont été reportées en position 2′ (ex. : $R_1$ = F), au niveau de l'atome d'azote (ex. : $R_2$, $R_3$ = alcoyle (éventuellement cyclique), acyle) et en position 4′ (ex. : $R_4$ = H, $R_5$ = OH, $R_4$ = $R_5$ = H, etc...).

En particulier, les dérivés désoxy-4′ ($R_4$ = $R_5$ = H, $R_1$ = $R_2$ = $R_3$ = H) 3 (R = H) ou 4 (R = OH) apparais sent aussi actifs *in vivo* chez la souris que les produits correspondants (daunorubicine 2a et doxorubicine 2b) pour la leucémie L1210, le sarcome S 180 et la leucémie P 388.

De plus, ces dérivés apparaissent moins cardiotoxiques dans les modèles animaux que 2a et 2b, ce qui apparaît intéressant puisque l'utilisation clinique des anthracyclines est souvent limitée par cet effet secondaire (F. ARCAMONE, Ref. op. cité, page 211-213).

La synthèse d'amino désoxy sucres homologues a été proposée par K.X. CHEN, H. GRESCH et B. PULLMAN (Molecular Pharmacology, **30,** 279, 1986) à partir de modèles d'intercalation de la daunorubicine 2a avec un hexanucléotide. Cet auteur conclut de cette étude théorique, que le remplacement du groupe $-NH_2$ de la daunosamine par un groupement $-CH_2NH_2$ doit apporter une plus grande stabilité au complexe ADN-anthracycline, stabilité qui est corrélée à l'activité démontrée par les anthracyclines.

C'est pourquoi la Demanderesse, poursuivant l'étude entreprise concernant la synthèse de nouveaux glycosides de grande valeur thérapeutique (cf. notamment Brevets français n° 84 03634 et n° 85 10063) a mis au point un procédé de synthèse d'un amino désoxy sucre homologue permettant d'obtenir des anthracyclines présentant une activité cytotoxique intéressante en thérapeutique, lesdites anthracyclines étant très stables.

La présente invention s'est, en conséquence, donné pour but de fournir de nouvelles anthracyclines qui répondent mieux aux nécessités de la pratique en raison de leur très grande stabilité ainsi que de nouveaux sucres servant à leur préparation.

La présente invention a pour objet des analogues de la désoxy-4 daunosamine, correspondant à la

formule générale 1 :

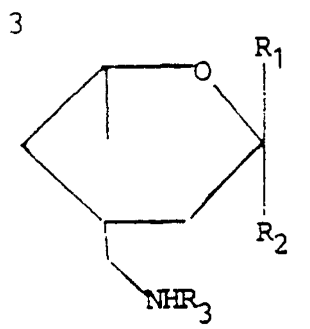

(1)

dans laquelle :

$R_1$ et $R_2$ sont toujours différents et représentent un atome d'hydrogène, un groupe hydroxyle, un groupe alcoxy ou un groupe acyloxy, à condition que lorsque l'un de ces groupements, $R_1$ ou $R_2$, représente un groupe hydroxyle, alcoxy ou acyloxy, l'autre groupement représente un atome d'hydrogène,

étant donné que lorsque $R_2$ est un atome d'hydrogène, on obtient un stéréoisomère $\alpha$ et lorsque $R_1$ est un atome d'hydrogène, on obtient un stéréoisomère $\beta$.

$R_3$ représente un atome d'hydrogène ou un groupe $COR_4$, $R_4$ représentant un groupe alkyle éventuellement substitué ou un groupe aryle éventuellement substitué ou un groupe perfluoroalkyle.

La présente invention a également pour objet un procédé de préparation des analogues de la désoxy-4 daunosamine de formule 1, caractérisé en ce que :

a - l'on condense l'oxyde de propylène (S) de formule 5 avec l'anion du cyano-3 diéthoxy-1,1 propane de formule 6, ladite condensation fournissant un mélange de deux nitriles de formule 7a et 7b ;

b - l'on cyclise le mélange obtenu au cours de l'étape a -, pour obtenir un mélange de 4 isomères de formule 8a, 9a, 8b, 9b ;

c - l'on sépare par chromatographie lesdits isomères ;

d - l'on épimérise en milieu basique les isomères axiaux de formule 8b et 9b pour obtenir les isomères équatoriaux 8a, 9a recherchés et ainsi recycler les iso mères 8b et 9b, selon le schéma I suivant :

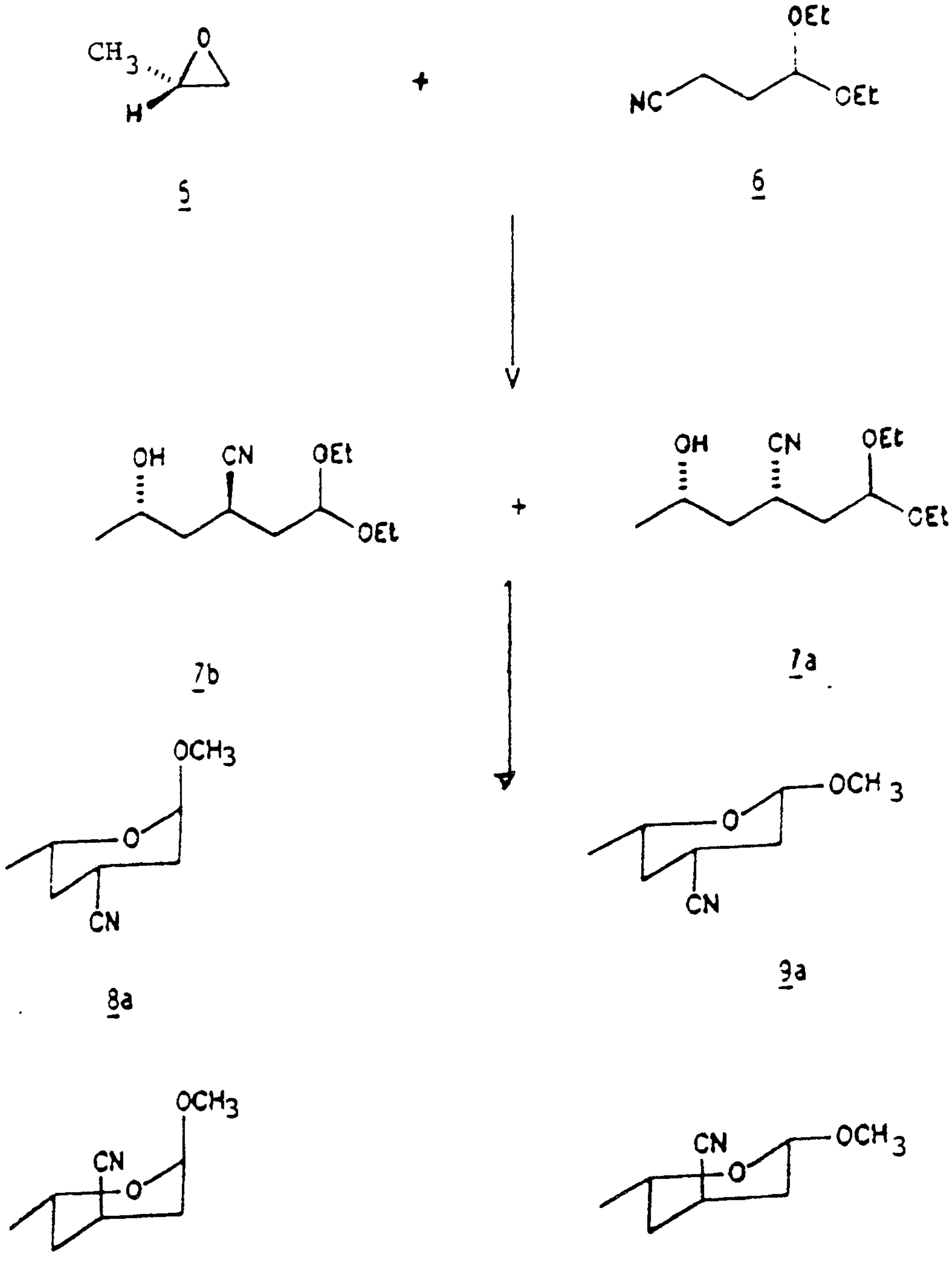

5 6 7b 7a 8a 9a 8b 9b

Schéma I

e - l'on réduit l'isomère 8a de manière à obtenir un produit de formule 10 comportant une fonction amine primaire ;

f - l'on protège la fonction amine primaire dudit composé de formule 10 par un traitement par un composé de formule $CF_3$-COO-$R_5$, dans laquelle $R_5$ est un groupe alkyle, pour obtenir le composé de formule 1a ;

g - l'on hydrolyse la fonction acétal du composé de formule 1a, pour donner un mélange de deux anomères α et β de formule 1b porteurs d'une fonction hémiacétal,

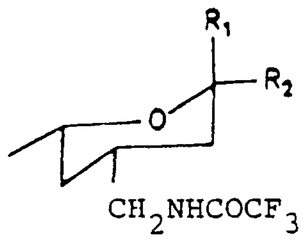

1b (α et ß),

dans laquelle $R_1$ et $R_2$ ont les mêmes significations que précédemment ;

h - l'on estérifie la fonction alcool du mélange de produits 1b pour obtenir le composé de formule 1c

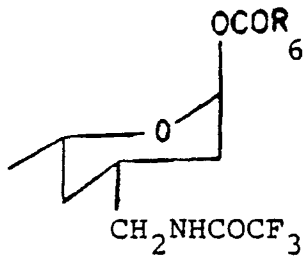

1c

dans laquelle $R_6$ représente un groupe alkyle éventuellement substitué ou un groupe benzyle éventuellement substitué, selon le schéma II suivant :

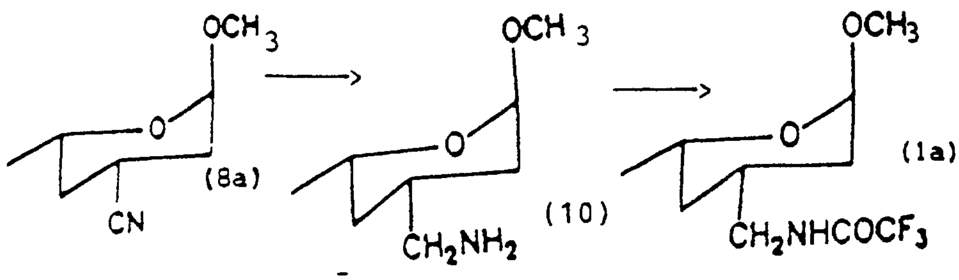

(8a) (10) (1a)

schéma II

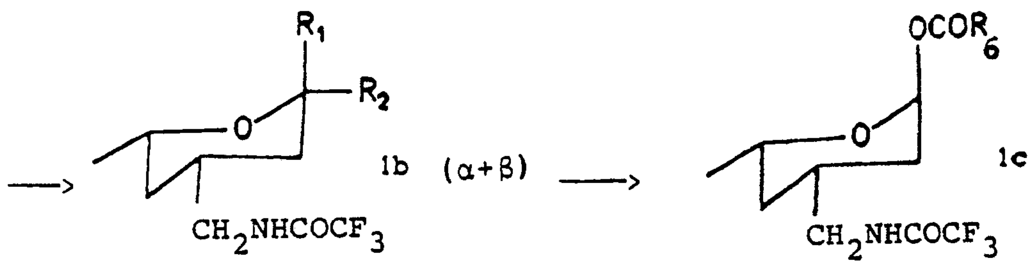

1b (α+β) 1c

La présente invention a également pour objet un procédé de préparation de nouvelles anthracyclines, à partir d'un analogue de la désoxy-4 daunosamine selon l'invention, caractérisé en ce qu'un composé de formule 1 est couplé à une anthracyclinone appropriée par une réaction acidocatalysée.

Selon un mode de mise en oeuvre dudit procédé, un produit de formule 1c est couplé à une anthracyclinone appropriée en présence d'un acide fort.

Selon une disposition avantageuse de ce mode de mise en oeuvre, l'acide fort est un acide halogéné, les halogénures obtenus étant traités par un sel de métal lourd.

Selon un autre mode de mise en oeuvre, un produit de formule 1c est couplé à une anthracyclinone en présence de trifluorométhanesulfonate de triméthyl-silyle.

Selon encore un autre mode de mise en oeuvre, un produit de formule 1a est couplé à une

anthracyclinone en présence d'un acide fort.

Conformément à l'invention, les anthracyclinones sont choisies dans le groupe qui comprend la daunomycinone, la β-rhodomycinone, l'Σ-isorhodomycinone.

Lorsqu'un composé de formule 1a est couplé à la daunomycinone, on obtient un produit de formule 15a :

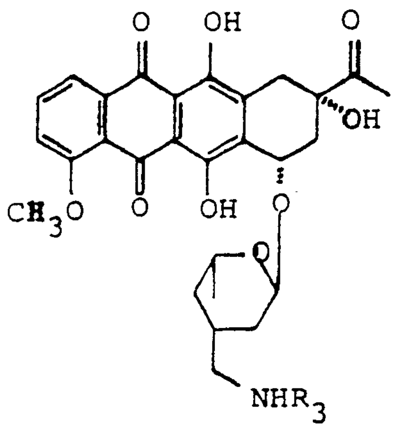

15a

dans laquelle :

$R_3$ est un groupe $COCF_3$.

Lorsqu'un composé de formule 1a est couplé avec la β-rhodomycinone, on obtient un composé de formule 16a :

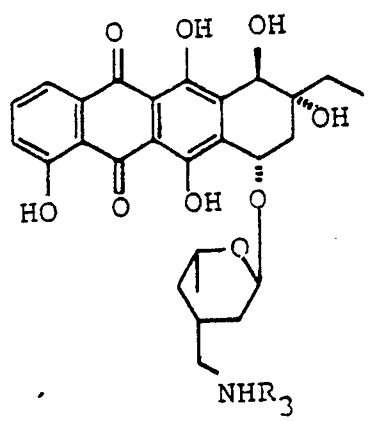

16a

dans laquelle :

$R_3$ est tel que défini ci-dessus.

Lorsqu'un composé de formule 1a est couplé avec l'Σ-isorhodomycinone, on obtient un composé de for mule 17a :

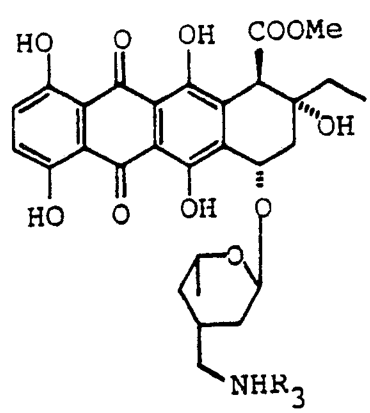

17a

dans laquelle :
$R_3$ est tel que défini ci-dessus.

Conformément à l'invention, les composés 15b, 16b et 17b sont obtenus à partir des composés 15a, 16a et 17a respectivement par hydrolyse en milieu basique.

Les glycosides obtenus sont purifiés par chromatographie.

Outre les dispositions qui précèdent, l'invention comprend encore d'autres dispositions qui ressortiront de la description qui va suivre.

L'invention vise plus particulièrement des analogues de la désoxy-4 daunosamine ainsi que de nouvelles anthracyclines et leurs procédés de préparation, ainsi que des compositions, en particulier des compositions thérapeutiques, dans lesquelles entrent lesdites anthracyclines, pour leur utilisation en chimiothérapie anti-cancéreuse.

Les nouvelles anthracyclines ont des propriétés cytotoxiques et antitumorales remarquables comme précisé dans les tests pharmacologiques, ci-après.

Parmi les différentes anthracyclines conformes à l'invention, présentant des résultats pharmacologiques très intéressants, il y a lieu de citer, notamment, le composé de formule 15a, le composé de formule 16a, le composé de formule 17a, le composé de formule 15b, le composé de formule 16b et le composé de formule 17b.

L'invention sera mieux comprise à l'aide du complément de description qui va suivre, qui se réfère à des exemples de mise en oeuvre du procédé, objet de la présente invention, ainsi qu'à des exemples mettant en évidence les propriétés pharmacologiques desdites anthracyclines.

**Exemple 1** : Synthèse du N-trifluoroacétyl aminométhyl-3 tetradésoxy-2,3,4,6-L-lyxo hexopyrannose de formule 1a.

Elle se caractérise en ce qu'elle implique quatre étapes pour obtenir le composé protégé 1a à partir de l'oxyde de propylène S 5 et du cyano-3 diéthoxy-1,1 propane 6.

**. Première étape :**

Elle implique la condensation avec l'époxyde (S) 5 de l'anion du nitrile 6 que l'on prépare avec une base forte (ex : LDA : diisopropylamidure de lithium) dans un solvant anhydre comme le THF (tétrahydrofuranne).

On obtient ainsi un mélange inséparable des nitriles 7a et 7b dans un rapport voisin de 1,5, comme suit :

A une solution, sous azote et à -10° C de diisopropylamine (4,6 ml, 3,28 $10^{-2}$ mole) dans le THF anhydre (32,4 ml), on ajoute goutte à goutte le n-BuLi (20,5 ml d'une solution 1,6 M dans l'hexane). Le milieu réactionnel incolore est maintenu à -10° C sous agitation pendant 1/2 heure, puis refroidi à -78° C.

Le cyanopropionaldéhyde diéthylacétal (4,32 g ; 2,75 $10^{-2}$ mole) en solution dans le THF anhydre (2 ml) est additionné et l'agitation maintenue pendant 2 heures à -78° C.

L'oxyde de propylène (2,04 g ; 1,3 éq.) est alors ajouté et le mélange réactionnel est maintenu sous agitation pendant 12 heures en laissant remonter doucement le milieu à température ambiante.

Après hydrolyse avec une solution de $NH_4Cl$, extraction à l'éther et séchage sur $Na_2SO_4$, on obtient après évaporation du solvant une huile jaune qui est chromatographiée rapidement sur gel de silice (éluant : Hexane AcOEt 70/30). Le mélange 7a,b est obtenu avec un rendement de 98 %.

* CYANO-3 DIETHOXY-1,1 HEXANOL-5(S) 7a + 7b :
RMN ($CDCl_3$) : 1,23 (t, 9H) ; 1,68 (m, 2H) ; 1,89 (m, 2H) 2,28 (m, 1H) ; 2,89 (p., J = 6,5 Hz, 0,5 H) ; 3,05 (h., J = 4,5 Hz, 0,5 H) ; 3,65 (m., 4H) ; 4,01 (m, 1H) ; 4,70 (t, 1H).

**. Deuxième étape : cyclisation en milieu acide de 7a,b :**

Le mélange de nitriles précédents (7a et 7b) est traité en milieu méthanolique acide, ce qui conduit aux quatre composés 8a, 8b, 9a, 9b qui peuvent être isolés après chromatographie sur silice.

Le rapport des composés 8a + 9a/8b + 9b est identique à celui des composés 7a et 7b (≈ 1,5). Les anomères les plus stables 8a et 8b représentent environ 80 % du mélange.

Une équilibration des composés 8a et 8b peut être réalisée en présence de tertiobutylate de potassium

dans le tertiobutanol ou dans tout autre milieu basique approprié. On obtient ainsi un rapport 8a/8b voisin de 80/20. La même réaction peut être effectuée sur le composé anomère β ainsi que sur le mélange des quatre produits 8a,b et 9a,b.

A une solution du mélange 7a + 7b (2,55 g ; 1,18 $10^{-2}$ mole) dans 12,5 ml de méthanol anhydre est ajoutée à température ambiante et sous atmosphère de $N_2$, 13 ml d'une solution de HCl 0,92 M dans le MeOH. Après 16 heures de temps de réaction, un contrôle en CCM (éluant : Hexane/ AcOEt 80/20) indique la disparition du produit de départ. Après hydrolyse, une extraction par de l'éther (lavage avec $H_2O$-$NaHCO_3$, eau, séchage sur $Na_2SO_4$) donne un brut réactionnel qui est chromatographié sur gel de silice. Une élution progressive (éluant : hexane/AcOEt 98/2 ; 95/5, 90/10, 80/20) permet d'isoler chacun des isomères par ordre de polarité croissante 8a (32 %) sous forme d'huile, 9b (19 %) sous forme d'huile, 9a (7,5 %) sous forme d'huile et 8b (41 %) cristallisé blanc, F = 61 °C. Le rendement de la réaction de cyclisation est de 82 %.

* METHYL CYANO-3 TETRADESOXY -2,3,4,6, α-L-LYXO HEXOPYRANOSIDE 8a :
RMN ($CDCl_3$) : 1,18 (d, J = 7 Hz, 3H) ; 1,53 (q, J = 11,5 Hz, 1H) ; 1,82 (t.d, $J_1$ = 11,5 Hz, $J_2$ = 3,5 Hz, 1H) ; 2 (m, 2H) ; 3,9 (tt, $J_1$ = 3,5 Hz, $J_2$ = 11,5 Hz, 1H) ; 3,33 (s, 3H) ; 3,82 (m, 1H) ; 4,76 (s large, $W_H$ = 6 Hz, 1H).
I.R. ($CHCl_3$) : 3020, 2980, 2940, 2840, 2220, 1440, 1385, 1320, 1210, 1170, 1150, 1110, 1080, 1040, 1000, 985, 975, 960, 910, 900, 860, 750.
$\alpha_D$ = -126,3° (c = 0,65, $CHCl_3$).

* METHYL CYANO-3 TETRADESOXY-2,3,4,6 β-L-RIBO HEXOPYRANOSIDE 9b :
RMN ($CDCl_3$) : 1,3 (d, J = 6 Hz, 3H) ; 1,36 (m, 2H) ; 1,84 (dd, $J_1$ = 9 Hz, $J_2$ < 2 Hz, 1H) ; 2,04 (dd, $J_1$ = 9 Hz, $J_2$ < 2 Hz, 1H) ; 3,17 (s large, $W_H$ = 10 Hz, 1H) ; 3,52 (s, 3H) ; 3,92 (s.d, $J_1$ = 7,5 Hz, $J_2$ ≈ 2 Hz, 1H) ; 4,63 et 4,87 (d.d, $J_1$ = 9,5 Hz, $J_2$ = 2,5 Hz, 1H).
I.R. ($CHCl_3$) : 3020, 2980, 2940, 2880, 2860, 2240, 1445, 1400, 1325, 1240, 1200, 1180, 1160, 1120, 1085, 1040, 1010, 990, 970, 870.

* METHYL CYANO-3 TETRADESOXY-2,3,4,6 β-L-LYXO HEXOPYRANOSIDE 9a :
RMN ($CDCl_3$) : 1,28 (d, J=6 Hz, 3H) ; 1,44-1,61 (m, 2H) ; 1,95 (md, J = 12,5 Hz, 1H) ; 2,05 (md, J = 12,5 Hz, 1H) ; 2,75 (tt, $J_1$ = 5 Hz, $J_2$ = 12 Hz, 1H) ; 3,50 (s, 3H) ; 4,27-4,32 (dd, $J_1$ = 9 Hz, $J_2$ = 2,2 Hz, 1H).
I.R. ($CHCl_3$) : 3020, 2980, 2940, 2880, 2860, 2240, 1445, 1400, 1325, 1240, 1200, 1180, 1160, 1120, 1040, 1010, 990, 970, 870.

* METHYL CYANO-3 TETRADESOXY-2,3,4,6 α-L-RIBO HEXOPYRANOSIDE 8b :
RMN ($CDCl_3$) : 1,21 (d, J = 6 Hz, 3H) ; 1,56 (m, 1H) ; 1,81 (m, 2H) ; 2 (m, 1H) ; 3,03 (s large, $W_H$ = 11,5 Hz, 1H) ; 3,38 (s, 3H) ; 4,18 (sd,$J_1$ = 6 Hz, $J_2$ ≈ 2 Hz, 1H) ; 4,76 (s large, $W_H$= 8,8 Hz, 1H).
I.R. ($CHCl_3$) : 3020, 2980, 2940, 2920, 2850, 2260, 1440, 1385, 1370, 1360, 1320, 1300, 1250, 1185, 1120, 1040, 970, 890, 860.
$\alpha_D$ = -152° (c = 1,19, $CHCl_3$).
F = 61 °C.

EPIMERISATION DU COMPOSE 8b :

L'isomère 8b (0,126 g ; 8,16 $10^{-4}$ mole) en solution dans 2 ml de terbutanol est traité par du terbutylate de potassium (0,4 eq., 0,036 g). La réaction a été suivie en CLHP (Lichrosorb Si 60 µm, éluant : Hexane-AcOEt 30 %).

Les résultats sont regroupés dans le tableau ci-dessous :

| CONDITIONS DE REACTIONS | 8 b | 8 a |
|---|---|---|
| 12 h Température ambiante | 18,5 % | 81,5 % |
| 72 h Température ambiante | 19 % | 81 % |
| 72 h à reflux | 18 % | 82 % |

L'isomère 8b peut donc être recyclé ; les composés 8a et 8b étant très différents en polarité, une chromatographie permet leur séparation.

. **Troisième étape :**

Une réduction du nitrile 8a par $LiAlH_4$ dans le THF anhydre fournit l'amine primaire 10. Cette étape peut être réalisée également avec d'autres réducteurs chimiques ou par l'hydrogène en présence d'un catalyseur comme $PtO_2$ ou Ni dans un solvant approprié.

A une suspension de $LiAlH_4$ (0,213 g) dans 8,4 ml de THF anhydre on additionne goutte à goutte l'isomère 8a (0,827 g ; 5,33 $10^{-3}$ moles) en solution dans 16,4 ml de THF. Après 4 heures d'agitation à température ordinaire, un contrôle CCM indique la disparition complète du produit de départ. L'hydrolyse est effectuée en ajoutant doucement 1,3 ml d'une solution de KOH à 10 %. Le précipité d'aluminate de lithium formé est filtré et lavé plusieurs fois avec de l'acétate d'éthyle. Après évaporation des solvants, l'amine 10, huile jaune est obtenue avec un rendement de 91 %. Le composé 10 est utilisé sans purification.

* METHYL AMINOMETHYL-3 TETRADESOXY-2,3,4,6 α-L-LYXO HEXOPYRANOSIDE 10 :
RMN ($CDCl_3$) : 0,90 (q., J = 12 Hz, 1H) ; 1,17 (d, J = 7 Hz, 3H) ; 1,31 (m, 2H) ; 1,8 (m, 3H) ; 2,51-2,55 (dd, $J_1$ = 7 Hz, $J_2$ < 2 Hz, 2H) ; 3,35 (s, 3H) ; 3,85 (m, 1H) ; 4,77 (s large, $W_H$ = 7 Hz, 1H).

. **Quatrième étape :**

L'amine 10 (0,77 g; 4,84 $10^{-3}$ mole) est mise en solution dans le trifluoroacétate de méthyle (27 ml, 55 eq.) en présence de DBU (0,26 ml, 0,35 eq.).

Le mélange est agité une nuit à température ambiante. Après évaporation sous pression réduite du solvant, le résidu est repris plusieurs fois par du $CH_2Cl_2$ et évaporé à chaque fois. Le brut réactionnel obtenu est chromatographié sur gel de silice (éluant : $CH_2Cl_2$, MeOH 3 %). Le composé 1a (0,94 g) est isolé sous forme de cristaux blancs avec un rendement de 76 %.

* METHYL N-TRIFLUOROACETYL AMINOMETHYL-3 TETRADESOXY-2,3, 4,6 α-L-LYXO HEXOPYRANOSIDE DE FORMULE 1a :
RMN ($CDCl_3$) : 1,01 (q, J = 11,5 Hz, 1H) ; 1,19 (d, J = 7 Hz, 3H) ; 1,29 (td, $J_1$ ≈ 3 Hz,$J_2$ = 11,5 Hz, 1H) ; 1,70 (m, 2H) ; 2,20 (s large, $W_H$ = 23 Hz, 1H) ; 3,20 (t, J = 6 Hz, 2H) ; 3,34 (s, 3H) ; 3,85 (sept., J = 6 Hz, 1H) ; 4,77 (s large, $W_H$ = 7 Hz, 1H) ; 7,21 (s large, $W_H$ = 14 Hz, 1H).
I.R. ($CHCl_3$) : 3440, 3020, 2940, 2980, 2400, 1720, 1540, 1450, 1385, 1225, 1160, 1120, 1050, 980, 930, 750, 660.
$\alpha_D$ = -90,4° (c = 0,5, $CHCl_3$)
F = 67°C

**Exemple 2** : Synthèse du mélange de composés 1b(α + β) (N-trifluoroacétyl aminométhyl-3 (α et β)-L-lyxo hexopyranose) :

Une solution de composé 1a (exemple 1) (0,275 g ; 1,08 $10^{-3}$ mole) dans un mélange d'acide acétique pur (27 ml) et d'eau (45 ml) est chauffée à reflux durant 24 heures avec agitation puis évaporée à sec sous pression réduite. Le résidu est repris par de l'eau puis par de l'acétone et évaporé sous pression réduite à chaque fois.

Le mélange des alcools anomères α et β est obtenu sous forme de cristaux blancs avec un rendement de 95 %.

ALCOOL ANOMERE 1bα :

RMN (acétone) : 0,88 (q, J = 13 Hz, 1H) ; 1,11 (d, J = 6 Hz, 3H) ; 1,31 (m, 1H) ; 1,65 (m, 2H) ; 3,23 (t, J = 6,5 Hz, 2H) ; 4,05 (m, 1H) ; 5 (s large, $W_H$, = 5 Hz, 1H) ; 5,29 (s large, $W_H$ = 7 Hz, 1H) ; 8,54 (s large, $W_H$ = 20 Hz, 1H).

On note dans le spectre RMN la présence de signaux minoritaires provenant de l'anomère β représen tant environ 20 % du mélange.
$\alpha_D$ à l'équilibre : - 12,4° (c = 0,54, $H_2O$)

**Exemple 3** : Préparation de O-acétyl N-trifluoroacétyl aminométhyl-3 tétradésoxy-2,3 4,6 α-L-lyxo hexopyranose 1d :

Le mélange 1b(α) + 1b(β) (0,074 g ; 3,07 $10^{-4}$ mole) est traité par 1 ml d'anhydride acétique et par une trace d'$H_2SO_4$.

Après 48 heures d'agitation à température ambiante, l'hydrolyse de la manière habituelle conduit à un résidu (50 mg) dont le spectre RMN indique qu'il s'agit majoritairement de l'acétate 1d.

Une chromatographie (éluant : $CH_2Cl_2$ MeOH 2%) sur Florisil fournit l'acétate 1d (30 %) à côté des composés 1a et l'alcool de départ.

* O-ACETYL N-TRIFLUOROACETYL AMINOMETHYL-3 TETRADESOXY-2,3, 4,6 α-L-LYXO HEXOPYRANOSE 1d :

RMN ($CDCl_3$) : 1,01 (q, J = 13,5 Hz, 1H) ; 1,21 (d, J = 6 Hz, 3H) ; 1,75 (m, 2H) ; 2,90 (s, 3H) ; 3,26 (m, 2H) ; 3,94 (m, 1H) ; 6,21 (s large, $W_H$ = 6 Hz, 1H) ; 6,59 (s large, $W_H$ = 20 Hz, 1H).

**Exemple 4** : Préparation de O-paranitrobenzoyl N-trifluoroacétyl aminométhyl-3 tétradésoxy-2,3,4,6 α-L-lyxo hexopyranose 1e :

Le mélange d'alcools 1b(α) + 1b(β) (0,063 g ; 2,6 $10^{-4}$ mole) est mis en solution dans la pyridine (0,3 ml) en présence de 48,4 mg de chlorure de p-nitrobenzoyle et d'une trace de diméthylaminopyridine.

Après 5 jours de temps de réaction, un très faible avancement de la réaction est observée en CCM. Après hydrolyse et extraction, on isole le p-nitrobenzoate 1e (Rdt = 8 %).

* O-PARANITROBENZOYL N-TRIFLUOROACETYL AMINOMETHYL-3 TETRADESOXY-2,3,4,6 α-L-LYXO HEXOPYRANOSE 1e :

RMN ($CDCl_3$) : 1,22 (d, J = 6 Hz, 3H) ; 1,57 (td, $J_1$ = 12,5 Hz, $J_2$ = 3 Hz, 1H) ; 1,91 (m, 1H) ; 2,35 (s large, 1H) ; 3,35 (m, 2H) ; 4,06 (m, 1H) ; 6,51 (s large, $W_H$ = 7,5 Hz, 2H) ; 8,23 (m, 4H).

**Exemple 5** : Glycosidation de la β-rhodomycinone avec un composé de formule 1a :

L'aglycone (0,1 g ; 2,59 $10^{-4}$ mole) est mis en solution dans le $CH_2Cl_2$ anhydre (40 ml) en présence de tamis moléculaire 4 Å (0,1 g) et d'acide paratoluènesulfonique anhydre (0,1 éq., 4,9 mg). Le milieu réactionnel est agité sous atmosphère d'azote pendant 1/2 heure.

Une solution du O-méthyl sucre (0,066 g, 1 éq.) dans le $CH_2Cl_2$ (2 ml) est additionnée et la réaction est suivie en CCM (éluant : $CH_2Cl_2$, MeOH 3 %).

Après 3 heures à température ambiante, la quantité d'APTS est augmentée (jusqu'à 0,5 éq.) et l'agitation poursuivie pendant une nuit.

Après hydrolyse et extraction avec $CH_2Cl_2$, l'ensemble des phases organiques est lavé avec une solution de bicarbonate de sodium, puis avec $H_2O$ jusqu'à neutralité et séché sur $Na_2SO_4$. Le mélange aglycone-glycoside obtenu après évaporation du solvant est séparé sur plaque préparative (1-éluant : $CH_2Cl_2$ MeOH 3 %, 2-éluant : $CH_2Cl_2$). Le composé 16a est ainsi obtenu avec un rendement de 10-16 %.

* O-(N-TRIFLUOROACETYL AMINOMETHYL-3′ TETRADESOXY-2′,3′, 4′,6′-α-L-LYXO-HEXOPYRANOSYL)-7 β-RHODOMYCINONE 16a :

RMN ($CDCl_3$) : 1,11 (t, J = 7 Hz, 3H) ; 1,26 (d, J = 6 Hz, 3H) ; 3,19 (m, 2H) ; 4,16 (m, 1H) ; 4,87 (s, 1H) ; 5,14 (s, 1H) ; 5,47 (s, 1H); 6,53 (s large, J = 11 Hz, 1H) ; 7,30 (d, J = 6 Hz, 1H) ; 7,68 (t, J = 8 Hz, 1H) ; 7,78 (d, J = 6 Hz, 1H) ; 12 (s, 1H) ; 12,73 (s, 1H) ; 13,5 (s, 1H).

I.R. : 3680, 3600, 3520, 3440, 2940, 1725, 1620, 1580, 1550, 1460, 1440, 1410, 1390, 1370, 1290, 1265, 1230, 1200, 1165, 1025, 980.

$\alpha_D$ : 192,5° (c = 0,2, $CHCl_3$).

F = 95-97° C.

**Exemple 6** : Glycosidation de la daunomycinone avec un composé de formule 1a :

Le protocole est identique à celui de l'exemple 5. On obtient ainsi le composé 15a.

* O-(N-TRIFLUOROACETYL AMINOMETHYL-3′ TETRADESOXY-2′, 3′, 4′,6′-α-L-LYXO-HEXOPYRANOSYL)-7 DAUNOMYCINONE 15a :

RMN ($CDCl_3$) : 1,25 (m, 3H) ; 2,42 (s, 3H) ; 3 (d, J = 15 Hz, 1H) ; 3,23 (t, J = 7 Hz, 2H) ; 4,06 (s, 3H) ; 4,78 (s, 1H) ; 5,31 (s, 1H) ; 5,54 (s, 1H) ; 6,36 (s large, 1H) ; 7,41 (d, J = 8 Hz, 1H) ; 7,78 (t, J = 8 Hz, 1H)

; 8 (d, J = 8 Hz, 1H) ; 13,28 (s, 1H) ; 13,98 (s, 1H).
I.R. ($CH_2Cl_2$) : 3680, 3420, 3060, 2930, 1730, 1620, 1580, 1440, 1410, 1260, 1210, 1170, 985.
$\alpha_D$ : 206,9° (c = 0,16, $CHCl_3$).
F = 85-85° C.

**Exemple 7** : Glycosidation de l'Σ-isorhodomycinone avec un composé de formule 1a :

On procède comme dans l'exemple 5. On obtient ainsi le composé de formule 17a;
* O-(N-TRIFLUOROACETYL AMINOMETHYL-3′ TETRADESOXY-2′, 3′, 4′,6′-α-L-LYXO-HEXOPYRANOSYL)-7 Σ-ISORHODOMYCINONE 17a :
RMN ($CDCl_3$) : 1,12 (t, 3H) ; 1,25 (d, 3H) ; 3,21 (m, 2H) ; 3,7 (s, 3H) ; 4,11 (m, 1H) ; 4,57 (s, 1H) ; 5,25 (s, 1H) ; 5,49 (s, 1H) ; 6,31 (s large, 1H) ; 12,29 (s, 2H) ; 12,82 (s, 1H) ; 12,95 (s, 1H).
$\alpha_D$ : -140° (c = 0,11, $CHCl_3$)
F = 186° C

COMPTE RENDU PHARMACOLOGIQUE CONCERNANT DES GLYCOSIDES SELON L'INVENTION.

**TEST DE PROLIFERATION :**

A - Protocole opératoire (réduction MTT) :

Des cellules tumorales L 1210, A 549 et HT 29 en croissance exponentielle, à une densité de $5.10^3$/ml dans un milieu RPMI, sont incubées dans des plaques de microtitration contenant 96 puits, pendant 72 heures (37° C, 5 % $CO_2$, 95 % d'humidité relative) avec différentes concentrations de chacun des glycosides conformes à l'invention.

Les témoins consistent en cellules tumorales dans un milieu de culture frais.

Quatre puits sont préparés pour chaque concentration de glycoside et pour le témoin.

Après 65 heures, 50 µl de MTT (2,5 mg/ml dans du PBS) sont ajoutés.

Le MTT sera réduit, en présence de cellules vivantes, en un colorant formazan rouge insoluble.

Après 7 à 24 heures d'incubation supplémentaire, le surnageant est enlevé.

Le colorant formazan est solubilisé par l'addition de 100 µl de DMSO dans chaque puits, suivie d'une agitation douce. L'extinction est mesurée pour chaque puits, à 492 nm (photomètre Multiscan 340 cc, Fa. Flow).

B - Résultats :

Les résultats sont exprimés sous forme de relation de l'extinction après incubation avec les glycosides par rapport à l'extinction obtenue avec les témoins. Le coefficient de variation est inférieur à 15 %. Les résultats sont représentés dans le tableau I, ci-après :

| | TEST AU MTT | | |
|---|---|---|---|
| | L1210 | HT 29 | A 549 |
| 16a | 0,14 | 0,48 | 0,14 |
| 16b | 0,11 | 0,13 | 0,13 |
| 15a | 0,25 | 0,67 | 0.43 |
| 15b | 0,04 | 0,1 | 0,04 |
| $IC_{50}$ : µg/ml | | | |

Les médicaments contenant les anthracyclines conformes à la présente invention sont généralement administrés à des doses comprises entre 0,001 et 25 mg/kg/jour.

Ils sont particulièrement adaptés au traitement des leucémies aiguës et chroniques, à la maladie de Hodgkin, aux lymphomes non-hodgkiniens.

Ainsi que cela ressort de ce qui précède, l'invention ne se limite nullement à ceux de ces modes de réalisation, de mise en oeuvre et d'application qui viennent d'être décrits de façon plus explicite ; elle en embrasse au contraire toutes les variants qui peuvent venir à l'esprit du technicien en la matière, sans s'écarter du cadre ni de la portée de la présente invention.

## Revendications

1°) Analogues de la désoxy-4 daunosamine, correspondant à la formule générale 1 :

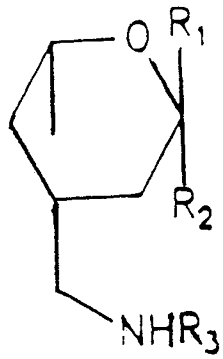

(1)

dans laquelle :

$R_1$ et $R_2$ sont toujours différents et représentent un atome d'hydrogène, un groupe hydroxyle, un groupe alcoxy, ou un groupe acyloxy à condition que lorsque l'un de groupements $R_1$ ou $R_2$ représente un groupe hydroxyle, alcoxy ou acyloxy, l'autre groupement représente un atome d'hydrogène,

étant donné que lorsque $R_2$ est un atome d'hydrogène, on obtient un stéréoisomère $\alpha$ et lorsque $R_1$ est un atome d'hydrogène, on obtient un stéréoisomère $\beta$.

$R_3$ représente un atome d'hydrogène ou un groupe $COR_4$, $R_4$ représentant un groupe alkyle éventuellement substitué ou un groupe aryle éventuellement substitué ou un groupe perfluoroalkyle.

2°) Procédé de préparation des analogues de la désoxy-4 daunosamine de formule 1, selon la revendication 1, caractérisé en ce que :

a - l'on condense l'oxyde de propylène (S) de formule 5 avec l'anion du cyano-3 diéthoxy-1,1 propane de formule 6, ladite condensation fournissant un mélange de deux nitriles de formule 7a et 7b ;

b - l'on cyclise le mélange obtenu au cours de l'étape a -, pour obtenir un mélange de 4 isomères de formule 8a, 9a, 8b, 9b ;

c - l'on sépare par chromatographie lesdits isomères ;

d - l'on épimérise en milieu basique les isomères axiaux de formule 8b et 9b pour obtenir les isomères équatoriaux 8a, 9a recherchés et ainsi recycler les isomères 8b et 9b, selon le schéma I suivant :

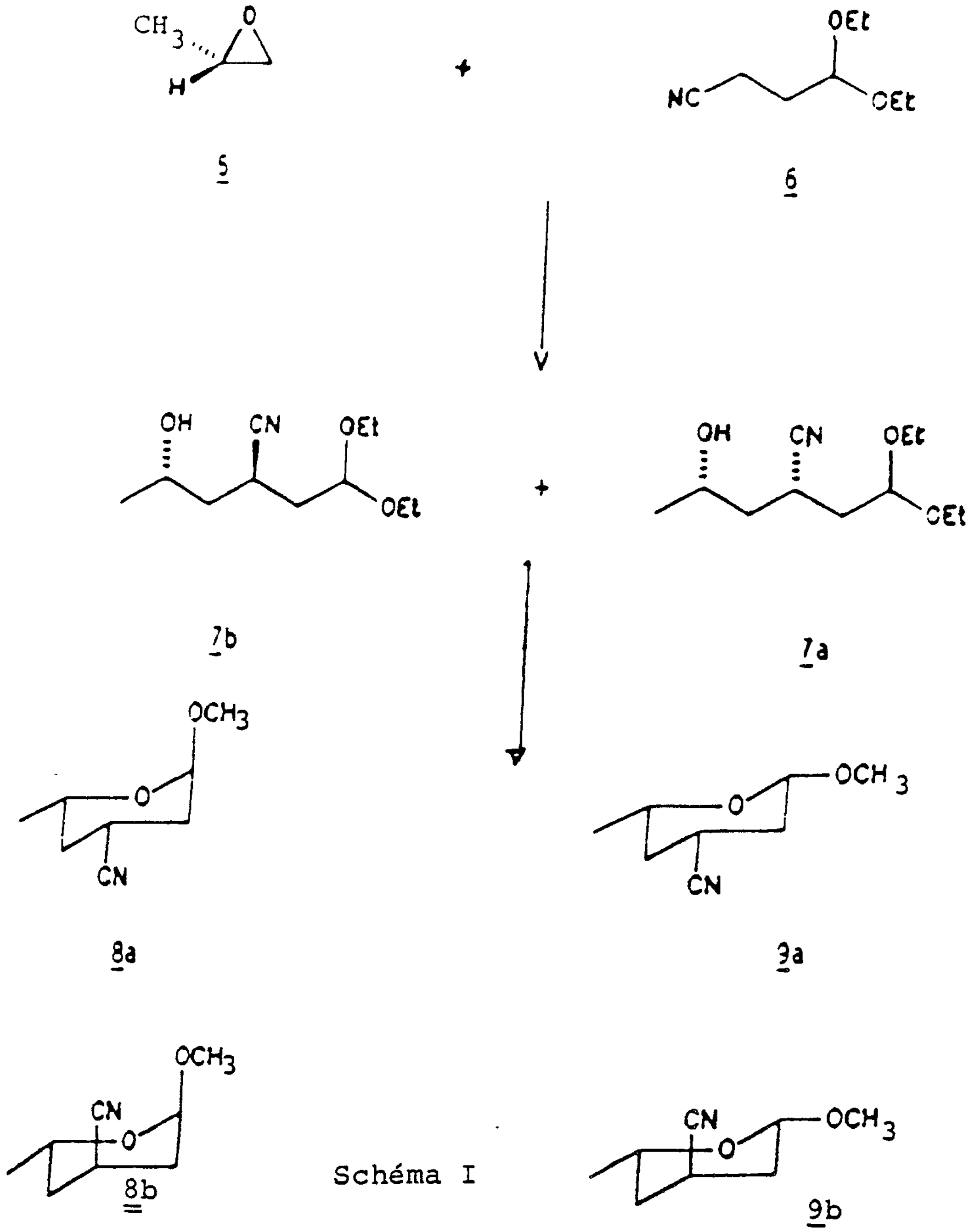

5 + 6

7b + 7a

8a 9a

8b Schéma I 9b

e - l'on réduit l'isomère 8a de manière à obtenir un produit de formule 10 comportant une fonction amine primaire;

f - l'on protège la fonction amine primaire dudit composé de formule 10 par un traitement par un composé de formule $CF_3$-COO-$R_5$, dans laquelle $R_5$ est un groupe alkyle, pour obtenir le composé de formule 1a ;

g - l'on hydrolyse la fonction acétal du composé de formule 1a, pour donner un mélange de deux anomères α et β de formule 1b porteurs d'une fonction hémiacétal,

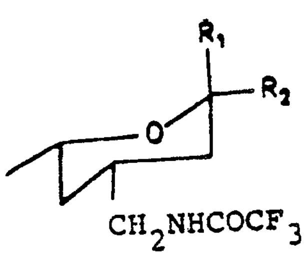

1b (α et ß),

dans laquelle $R_1$ et $R_2$ ont les mêmes significations que précédemment ;

h - l'on estérifie la fonction alcool du mélange de produits 1b pour obtenir le composé de formule 1c

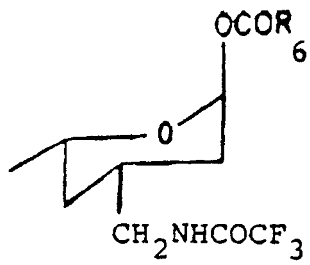

1c

dans laquelle $R_6$ représente un groupe alkyle éventuellement substitué ou un groupe benzyle éventuellement substitué, selon le schéma II suivant :

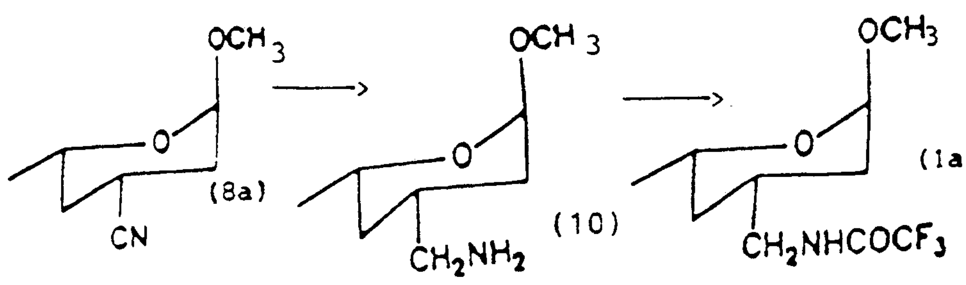

(8a) (10) (1a)

schéma II

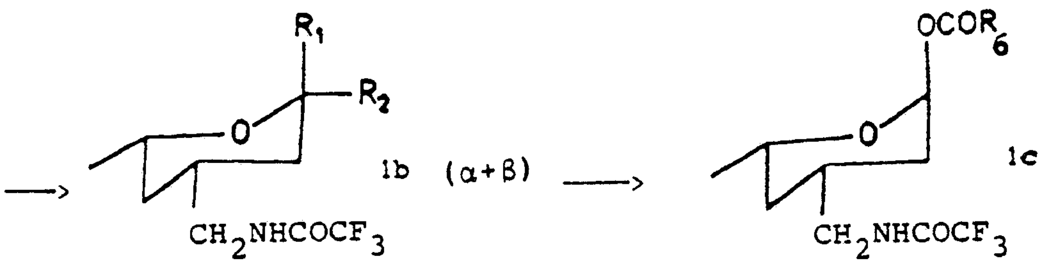

1b (α+β) 1c

3°) Procédé de préparation de nouvelles anthracyclines à partir d'un analogue de la désoxy-4 daunosamine selon la revendication 1, caractérisé en ce qu'un composé de formule 1 est couplé à une anthracyclinone appropriée par une réaction acidocatalysée.

4°) Procédé selon la revendication 3, caractérisé en ce qu'un produit de formule 1c est couplé à une anthracyclinone appropriée en présence d'un acide fort.

5°) Procédé selon la revendication 4, caractérisé en ce que l'acide fort est un acide halogéné, les halogénures obtenus étant traités par un sel de métal lourd.

6°) Procédé selon la revendication 3, caractérisé en ce qu'un produit de formule 1c est couplé à une anthracyclinone en présence de trifluorométhanesulfonate de triméthyl-silyle.

7°) Procédé selon la revendication 3, caractérisé en ce qu'un produit de formule 1a est couplé à une anthracyclinone en présence d'un acide fort.

8°) Procédé selon l'une quelconque des revendications 3 à 7, caractérisé en ce que les anthracyclinones sont choisies dans le groupe qui comprend la daunomycinone, la β-rhodomycinone, l'Σ-isorhodomycinone.

9°) Anthracycline, caractérisée en ce qu'elle est obtenue par le procédé selon la revendication 3 ou la revendication 7 et en ce qu'elle est de formule 15, ci-après :

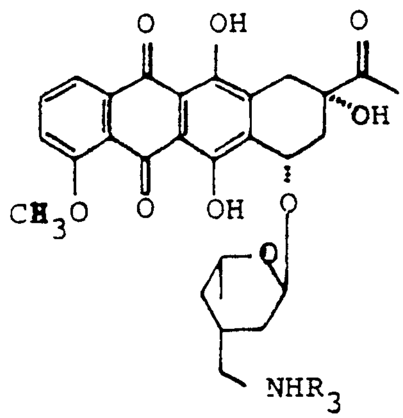

15

dans laquelle :

$R_3$ représente un groupe $COCF_3$ ou un atome d'hydrogène.

10°) Anthracycline, caractérisée en ce qu'elle est obtenue par le procédé selon la revendication 3 ou la revendication 7 et en ce qu'elle est de formule 16, ci-après :

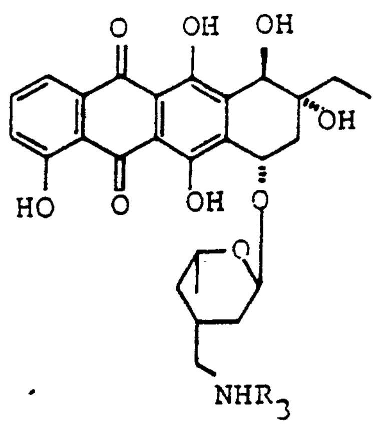

16

dans laquelle :

$R_3$ représente un groupe $COCF_3$ ou un atome d'hydrogène.

11°) Anthracycline, caractérisée en ce qu'elle est obtenue par le procédé selon la revendication 3 ou la revendication 7 et en ce qu'elle est de formule 17, ci-après :

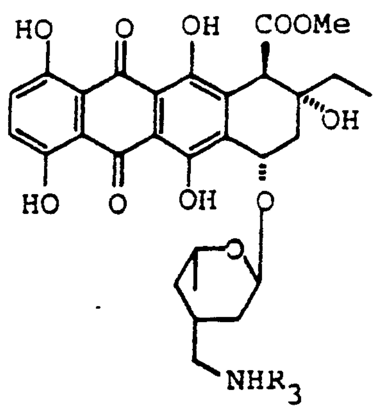

17

dans laquelle :

$R_3$ représente un groupe $COCF_3$ ou un atome d'hydrogène.

12°) Cyano-3 diéthoxy-1,1 hexanol-5(S) de formule 7a, ci-après :

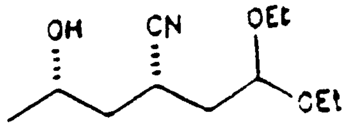

7a

produit intermédiaire du procédé selon la revendication 2.

13°) Cyano-3 diéthoxy-1,1 hexanol-5(S) de for mule 7b, ci-après :

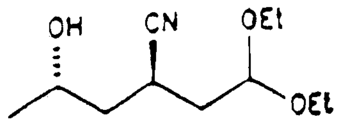

7b

produit intermédiaire du procédé selon la revendication 2.

14°) Méthyl cyano-3 tetradesoxy-2,3,4,6 α-L-lyxo hexopyranoside de formule 8a, ci-après :

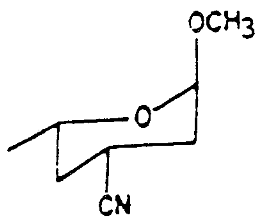

8a

produit intermédiaire du procédé selon la revendication 2.

15°) Méthyl cyano-3 tetradésoxy-2,3,4,6 β-L-ribohexopyranoside de formule 9b, ci-après :

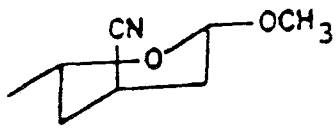

9b

produit intermédiaire du procédé selon la revendication 2.

16°) Méthyl cyano-3 tetradésoxy-2,3,4,6 β-L-lyxo hexopyranoside de formule 9a, ci-après :

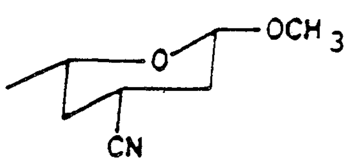

9a

produit intermédiaire du procédé selon la revendication 2.

17°) Méthyl cyano-3 tetradésoxy-2,3,4,6 α-L- ribohexopyranoside de formule 8b; ci-après :

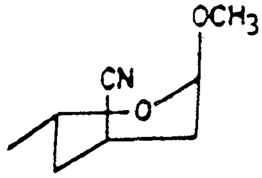

8b,

produit intermédiaire du procédé selon la revendication 2.

18°) Méthyl aminométhyl-3 tetradésoxy-2,3,4,6 α-L-lyxo hexopyranoside de formule 10, ci-après :

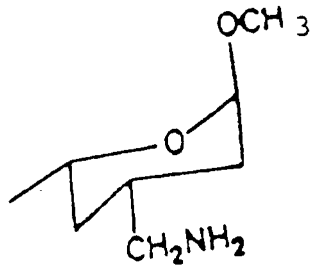

10

produit intermédiaire du procédé selon la revendication 2.

19°) Méthyl N-trifluoroacétyl aminométhyl-3 tétradesoxy-2,3,4,6 α-L-lyxo hexopyranoside de formule 1a, ci-après :

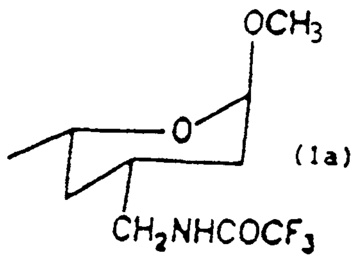

(1a)

1a

analogue de la désoxy-4 daunosamine, selon la revendication 1.

20°) N-trifluoroacétyl aminométhyl-3 α (et β)-L-lyxo hexopyranose de formule 1b, ci-après :

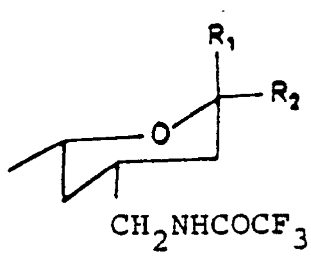

1b

analogue de la désoxy-4 daunosamine selon la revendication 1.

21°) O-acétyl N-trifluoroacétyl aminométhyl-3 tetradesoxy-2,3,4,6 α-L-lyxo hexopyranose de formule 1d, ci-après :

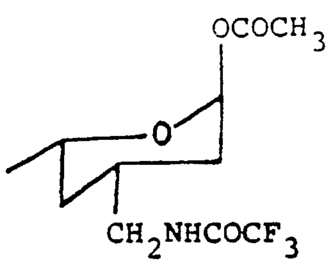

1d

analogue de la désoxy-4 daunosamine selon la revendication 1.

22°) O-paranitrobenzoyl N-trifluoroacétyl aminométhyl-3 tetradésoxy-2,3,4,6 α-L-lyxo hexopyranose de formule 1e, ci-après :

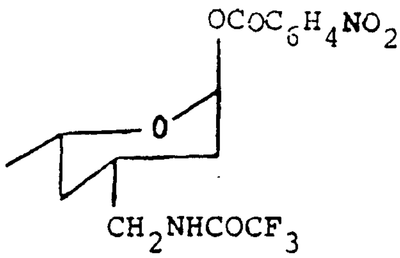

Ie

analogue de la désoxy-4 daunosamine selon la revendication 1.

23°) Composition pharmaceutique, caractérisée en ce qu'elle contient en tant que composé actif une ou plusieurs anthracyclines selon les revendications 9 à 11, éventuellement associées à au moins un véhicule pharmaceutiquement acceptable.

Office européen des brevets

# RAPPORT DE RECHERCHE EUROPEENNE

Numero de la demande

EP 89 40 2648

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | JOURNAL OF MEDICINAL CHEMISTRY, vol. 19, no. 12, décembre 1976, pages 1424-1425; F. ARCAMONE et al.: "Synthesis and antitumor activity of 4'-deoxydaunorubicin and 4'-deoxyadriamycin"<br>* En entier *<br>--- | 9-11,23 | C 07 H 15/252<br>C 07 D 309/10<br>C 07 C 255/13<br>A 61 K 31/70 |
| Y | FR-A-2 605 321 (FARMITALIA)<br>* Revendications 1,12,13 *<br>--- | 9-11,23 | |
| Y | CHEMICAL ABSTRACTS, vol. 105, no. 25, 22 décembre 1986, page 28, résumé no. 218418a, Columbus, Ohio, US; K. CHEN et al.: "A theoretical study of the comparative binding affinities of daunomycin derivatives to a double-stranded oligomeric DNA. Proposal for new high-affinity derivatives", & MOL. PHARMACOL. 1986, 30(3), 279-86<br>* Résumé *<br>----- | 9-11,23 | **DOMAINES TECHNIQUES RECHERCHES (Int. Cl.5)**<br>C 07 H 15/00<br>C 07 D 309/00<br>C 07 C 255/00<br>A 61 K 31/00 |

Le présent rapport a été établi pour toutes les revendications

| Lieu de la recherche | Date d'achèvement de la recherche | Examinateur |
|---|---|---|
| LA HAYE | 19-12-1989 | BRENNAN J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant